# EUROPÄISCHE PATENTSCHRIFT

(11) **EP 1 514 522 B1**
(45) Veröffentlichungstag und Bekanntmachung des Hinweises auf die Patenterteilung: **06.05.2009**
(21) Anmeldenummer: 04021636.8
(22) Anmeldetag: 10.09.2004
(51) Int. Cl.: A61B 17/70

(54) **Knochenschraube**
Bone screw
Vis osseuse

(30) Priorität: 12.09.2003 DE 20314297 U
(43) Veröffentlichungstag der Anmeldung: 16.03.2005
(73) Patentinhaber: Signus Medizintechnik GmbH, 63755 Alzenau (DE)
(72) Erfinder: Dierks, Michael, 42929 Wermelskirchen (DE)
(74) Vertreter: Gudat, Axel

(56) Entgegenhaltungen:
- DE-A- 19 950 075
- US-A- 5 569 247
- US-A- 5 716 357
- US-A- 6 004 322

## Beschreibung

Die Erfindung betrifft eine Knochenschraube zur Anbringung eines spinalen Fixiermittels, insbesondere als Pedikelschraube, mit einem in einem Knochen, insbesondere Wirbel, festlegbaren Schraubenschaft mit Gewinde zum Einschrauben in den Knochen und mit einem Schraubenkopf mit einem Befestigungsbereich für eine Befestigungseinrichtung, mittels derer das spinale Fixiermittel festlegbar ist, wobei der Befestigungsbereich einen Gewindeabschnitt zur Festlegung der Befestigungseinrichtung aufweist und wobei ferner eine Befestigungseinrichtung zur Festlegung eines spinalen Fixiermittels an der Knochenschraube umfasst ist.

Derartige Knochenschrauben sind vielfältig bekannt und können beispielsweise zur Stabilisierung oder Lagefixierung von Knochen zueinander verwendet werden, insbesondere zur Stabilisierung und Fixierung der Wirbelsäule, indem die jeweiligen Knochenschrauben in die zu fixierenden Knochen wie beispielsweise in Wirbelkörper eingeschraubt und mittels des an der zugeordneten Befestigungseinrichtung festgelegten Fixiermittels wie eines stabförmigen Längsträgers für die Osteosynthese befestigt werden.

Eine derartige Knochenschraube mit Befestigungseinrichtung ist aus der DE 199 44 120 A1 bekannt. Der Befestigungsbereich der Knochenschraube für die Befestigungseinrichtung ist in Form eines Kegelstumpfes gestaltet, der an einem teilkugelförmigen Schraubenkopf ausgebildet ist, wobei der sich zum Schraubenschaft hin vorzugsweise konisch erweiternde Kegelstumpf eine Gewindebohrung zur Aufnahme für eine Gewindeschraube aufweist, mittels welcher die als Klemmschelle ausgebildete Befestigungseinrichtung an dem Schraubenkopf festlegbar ist. Hierdurch soll ermöglicht werden, dass auch nach Platzierung der Knochenschraube die Lage des Längsträgers relativ zu deren Längsachse frei geändert werden kann.

Bei einer derartige Knochenschraube hat es sich jedoch als nachteilig herausgestellt, dass bei implantierter Knochenschraube aufgrund der abstehenden Kegelstümpfe eine sichere Anordnung und einfache Handhabung der Klemmschellen auf dem Kegelstumpf während der Festlegung und/oder der Ausrichtung des spinalen Fixiermittels nicht immer in der gewünschten Weise möglich ist. So ist bei dieser Anordnung die Schelle mit dem Schellenbogen, welcher der Aufnahme des Fixierstabes dient, sehr nahe an dem Dornfortsatz des Wirbelkörpers angeordnet. Ferner besteht aufgrund der vergleichsweise geringen Höhe des Kegelstumpfes bei einer Vormontierung der Schellen die Gefahr, dass diese von dem Kegelstumpf abrutschen. Des Weiteren ist es oftmals erforderlich, den Fixierstab zur Anpassung an die Solllage der Wirbelkörper zu verformen. Ein Einschieben des Verbindungsstabes in die entsprechenden Aufnahmen der Klemmschellen ist bei der bekannten Knochenschraube aufgrund der gegebenen Anordnung der Schellen jedoch teilweise recht umständlich. Andererseits ist das Anbringen des Verbindungsstabes insbesondere bei einer langstreckigen Instrumentierung über mehrere Wirbelkörper mit vormontierten Klemmschellen schwierig, da diese über die Kegelstümpfe der implantierten Knochenschrauben gehebelt werden müssen.

Darüber hinaus sind aus der DE 199 21 551 und der EP 641 548 Knochenschrauben bekannt, die eine oberflächliche Verzahnung zur Fixierung der Befestigungseinrichtung in verschiedenen Winkelstellungen ermöglichen. Die Verzahnung wird hierbei zumeist großflächig an einem gewölbten Kugelkopfabschnitt oder einer Kugelschnittfläche angebracht. Die Einbringung einer derartigen Verzahnung ist jedoch vergleichsweise aufwändig und kostenintensiv.

Eine Knochenschraube gemäß des Oberbegriffs von Anspruch 1 ist aus der US 5,569,247 bekannt.

Der Erfindung liegt die Aufgabe zugrunde, eine gattungsgemäße Knochenschraube derart weiterzubilden, dass das spinale Fixiermittel vergleichsweise einfach und sicher an der Knochenschraube festlegbar ist, auch unter Berücksichtigung einer Vormontierung der Befestigungseinrichtung an der Knochenschraube, und die gegebenenfalls auch minimalinvasiv implantierbar ist.

Die Aufgabe wird erfindungsgemäß durch eine Knochenschraube nach Anspruch 1 gelöst. Der Schraubenkopf weist eine von einem Gewindeabschnitt verschiedene Ausnehmung mit einer Längsachse aufweisen, die zur verschiebungssicheren Aufnahme eines Haltebereichs der Befestigungseinrichtung lateral zur Ausnehmungslängsachse ausgebildet ist, wobei die Ausnehmung so gestaltet ist, dass diese einen in die Ausnehmung eingreifenden, im Wesentlichen kongruent ausgebildeten Haltebereich der Befestigungseinrichtung lateral verschiebungssicher aufnehmen kann, wobei der Gewindeabschnitt sich zumindest im wesentlichen in Längsrichtung der Ausnehmung erstreckt.

In die Ausnehmung kann somit ein Fortsatz der Befestigungseinrichtung eingeführt werden, wodurch ein unbeabsichtigtes Abrutschen der Befestigungseinrichtung von dem Schraubenkopf auch bei schwierigen Handhabungen eines spinalen Fixiermittels wie bei einer Verbiegung desselben verhindert wird. Die Ausnehmung kann sich hierbei relativ weit in den Schraubenkopf hinein erstrecken, wodurch eine vergleichsweise große Anlagefläche der Befestigungseinrichtung an der Schraubenkopfinnenseite bereitgestellt werden kann, so dass sich auch in vormontiertem Zustand eine stabile Baugruppe ergibt und bei festgelegter Befestigungseinrichtung hohe Haltekräfte aufgenommen werden können. Ferner wird durch die erfindungsgemäße Ausbildung ermöglicht, die Außenkontur der Schraube als auch die Querschnittsgestaltungen der Ausnehmung und/oder des eingreifenden Haltebereichs unabhängig von anderen Erfordernissen an die jeweiligen Anforderungen optimal anzupassen, beispielsweise den Schraubenkopf weitestgehend als Kugelkopf auszubilden. Ferner ist die Knochenschraube mit Befestigungseinrichtung minimalinvasiv implantierbar.

Durch die Erfindung wird ferner eine Befestigungseinrichtung zur Zusammenwirkung mit einer Knochenschraube bereitgestellt, wobei der Befestigungsbereich der Einrichtung einen Fortsatz aufweist, der in die Ausnehmung der Knochenschraube eingreifen kann. Weitere vorteilhafte Ausführungen der Knochenschraube ergeben sich aus den Unteransprüchen.

Vorzugsweise kann die Ausnehmung den Haltebereich der Befestigungseinrichtung vollumfänglich lateral verschiebungssicher aufnehmen. Vorzugsweise ist die Ausnehmung vollumfänglich geschlossen, ohne hierauf beschränkt zu sein. Ferner ist die Befestigungseinrichtung im vormontiertem Zustand vorzugsweise vollumfänglich und stufenlos gegenüber dem Schraubenschaft verdrehbar.

Vorzugsweise ist die Ausnehmung des Schraubenkopf zur Aufnahme des Haltebereichs der Befestigungseinrichtung bzw. des Fortsatzes im Wesentlichen zylindrischen ausgeführt, worunter auch eine konische Ausbildung zu fassen ist, wobei der Gewindeabschnitt im Wesentlichen konzentrisch zu der Ausnehmung angeordnet ist.

Die Ausnehmung ist bezogen auf die Einführrichtung des Verbindungsmittels zur Festlegung der Befestigungseinrichtung dem Gewindeabschnitt vorgelagert und weist einen größeren Durchmesser auf als dieser. Hierdurch kann die Ausnehmung einen vergleichsweise großen Durchmesser aufweisen und die Ausnehmung und der Gewindeabschnitt, insbesondere wenn dieser als Gewindebohrung ausgeführt ist, können fertigungstechnisch einfach in den Schraubenkopf eingebracht werden. Ferner ist zur Festlegung der Befestigungseinrichtung zunächst lediglich das zugeordnete Verbindungsmittel in der Ausnehmung positionierbar, wodurch eine gewisse Vormontage erreicht wird, wobei erst anschließend das Verbindungsmittel an dem Gewindeabschnitt festgeschraubt wird.

Die Ausnehmung und der als Gewindebohrung ausgeführte Gewindeabschnitt sind in Art einer Stufenbohrung ausgeführt, die lediglich durch einen Absatz voneinander getrennt sind. Vorzugsweise weist der Absatz eine senkrecht zur Ausnehmungslängsachse verlaufende Oberseite auf, woran sich unmittelbar die Gewindebohrung anschließt. Gegebenenfalls können der Gewindeabschnitt und die Ausnehmung aber auch durch einen sich in Längsrichtung der Ausnehmung erstreckenden Übergangsbereich voneinander getrennt sein.

Vorteilhafterweise sind die Längsachsen des Gewindeabschnittes und der Ausnehmung jeweils geneigt zu der Schraubenschaftlängsachse angeordnet, und damit auch die vorzugsweise konzentrisch verlaufende Längsachse der Ausnehmung, wodurch sich eine einfache Handhabung der Knochenschraube ergibt. Der zwischen den Längsachsen von Gewindeabschnitt und Schraubenschaft eingeschlossene Winkel beträgt vorzugsweise bis zu 0°, beispielsweise ca. 30°-60° oder ca. 45°, ohne hierauf beschränkt zu sein. Gegebenenfalls kann die Gewindeabschnittslängsachse auch senkrecht zu der Schraubenschaftlängsachse angeordnet sein oder auch koaxial mit dieser.

Vorteilhafterweise verjüngt sich die Ausnehmung zum Inneren des Schraubenkopfes hin, wodurch sich der in die Ausnehmung eingesetzte Haltebereich der Befestigungseinrichtung selbst zentrieren kann und zum anderen ein Festsetzen in der Ausnehmung verhindert wird. Die Verjüngung kann konisch sein, die Wandung der Ausnehmung kann in ihrem Längsschnitt auch gebogen ausgeführt sein, wobei die Verjüngung sich mit zunehmendem Abstand zu der Einführöffnung der Ausnehmung zunehmend verjüngen kann.

Es hat sich als vorteilhaft erwiesen, wenn sich die Ausnehmung konisch mit einem Winkel von 1°-20° zwischen Wandung der Ausnehmung und Ausnehmungslängsachse verjüngt, vorzugsweise ca. 2° bis ca. 10-15°, beispielsweise ca. 7,5° ohne hierauf beschränkt zu sein.

Der die Ausnehmung und den Gewindeabstand radial außen umgebende Absatz kann in etwa die gleiche radiale Erstreckung aufweisen wie der die Ausnehmung radial außen umgebende Absatz, als besonders vorteilhaft hat es sich erwiesen, wenn der den Gewindeabschnitt radial außenseitig umgebende Absatz eine größere radiale Erstreckung aufweist als der die Ausnehmung radial außen umgebende Absatz, wodurch die Ausnehmung einen vergleichsweise großen Durchmesser im Bereich der Einführöffnung für den Haltebereich der Befestigungseinrichtung aufweist.

Vorzugsweise ist der Schraubenkopf auf Höhe zumindest eines Teilbereiches der Ausnehmung oder über die gesamte Höhe der Ausnehmung in Art einer Kugeloberfläche gewölbt. Hierdurch kann der Schraubenkopf im Bereich der Ausnehmung mit einer vergleichsweise großen Materialstärke bei gleichzeitig vergleichsweise großem Durchmesser der Ausnehmung ausgeführt sein, wodurch eine sehr stabile Befestigung des spinalen Fixiermittels und zugleich eine für die Einsetzung und Handhabung des Implantats günstige Geometrie des Schraubenkopfes gewährleistet ist.

Die Ausnehmung kann einen runden oder nichtrunden, insbesondere mehreckigen Querschnitt aufweisen, wobei Anlagebereiche für den in die Ausnehmung eingreifenden Fortsatz der Befestigungseinrichtung auf einem Kreisbogen liegen können. Der mehreckige Querschnitt kann insbesondere drei- bis zwölfeckig oder mehreckig, vorzugsweise fünf- bis achteckig ausgeführt sein. Vorzugsweise sind die Querschnitte der Anlagebereiche der Ausnehmung und des in diese eingreifenden Haltebereichs der Befestigungseinrichtung, welcher mit der Wandung der Ausnehmung zur Anlage kommt, derart angepasst, dass einer der Querschnitte rund und jeweils der andere Querschnitt nichtrund mit mindestens drei umfänglich verteilten Anlagebereichen ausgeführt ist, insbesondere mehreckig ausgeführt ist. Beispielsweise können der Querschnitt der Ausnehmung oder der Querschnitt des Haltebereichs der Befestigungseinrichtung rund ausgeführt sein, wobei der Querschnitt des jeweils korrespondierenden Bauteils nichtrund, insbesondere mehreckig, ausgeführt ist. Die Mehrecke können insbesondere regelmäßige Mehrecke sein. Hierdurch wird eine erleichterte Verdrehung der Befestigungseinrichtung um die Ausnehmungslängsachse bei an der Knochenschraube vormontierter Befestigungseinrichtung ermöglicht, ohne dass die gesamten Mantelflächen der Ausnehmungswandung bzw. des Haltebereichs der Befestigungseinrichtung miteinander zur Anlage kommen, als auch ein Festsetzen der Befestigungseinrichtung bei Ausübung hoher Klemmkräfte vermieden. Es versteht sich, dass von der Mehreckigkeit mehr oder weniger große Abweichungen möglich sind, beispielsweise in Form von abgerundeten Übergangsbereichen an den Ecken und/oder abgerundeten Zwischenbereichen, die zwischen den Anlagebereichen angeordnet sind. Die Anlagebereiche können somit linienförmig oder flächig ausgeführt sein.

Vorzugsweise erstreckt sich die Innenwandung der Ausnehmung in der Höhe auf der dem Schraubenschaft nächstbenachbarten Seite der Ausnehmung bis über die Verlängerung der Mantelfläche des Schraubenschaftes hinaus in den Schraubenkopf hinein. Die dem Schraubenschaft nächstbenachbarte Seite der Ausnehmung ergibt sich hierbei durch eine Ebene, die von der Ausnehmungslängsachse und der Schraubenschaftlängsachse aufgespannt wird, und durch die Neigungsrichtung der Ausnehmungslängsachse bestimmt wird. Die Mantelfläche des Schraubenschaftes kann die Einhüllende des Gewindeabschnitts oder die Einhüllende des Schaftbereichs, der durch die jeweilige Basis der Gewindeabschnitte bestimmt wird, definiert sein. Hierdurch kann die Ausnehmung eine große Tiefe aufweisen, ohne die Geometrie der Schraubenkopfoberfläche zu beeinflussen, die unabhängig von der Ausgestaltung der Ausnehmung oder deren Tiefe in geeigneter Weise gestaltet werden kann.

Vorzugsweise erstreckt sich die Ausnehmung derart in den Schraubenkopf hinein, dass der die Tiefe der Ausnehmung begrenzende Querschnitt bzw. der Boden der Ausnehmung in etwa auf Höhe oder auf der der Öffnung der Ausnehmung abgewandten Seite des Schnittpunktes von Schraubenschaftlängsachse und Ausnehmungslängsachse angeordnet ist. Der Boden der Ausnehmung kann hierbei durch den unteren Umfangsrand der Ausnehmung definiert werden, der Ausnehmungsboden kann selbstverständlich eine Öffnung aufweisen, an welche sich beispielsweise eine Gewindebohrung anschließen kann. Der oben definierte Schnittpunkt entspricht bei im Wesentlichen kugelförmiger Gestaltung des Schraubenkopfes dem Kugelmittelpunkt. Durch diese große Tiefe der Ausnehmung kann eine sehr sichere und stabile Vormontierung der Befestigungseinrichtung erfolgen und auch bei großen Haltekräften wird eine nur vergleichsweise geringe Flächenpressung der miteinander zur Anlage kommenden Anlagebereiche des Haltebereichs der Befestigungseinrichtung mit der Innenwandung der Ausnehmung erzielt.

Vorzugsweise ist der Schraubenkopf im Wesentlichen als Kugelkopf oder Teilkugelkopf ausgeführt, wobei der Kugelkopf vorzugsweise bis auf seitliche, einander gegenüberliegende Abplattungen und sonstigen Haltebereichen zur Befestigung von Einrichtungen, die für das jeweilige Implantat von Bedeutung sind, wie von Verlängerungsstäben oder dergleichen, kugelförmig ausgeführt ist. Die kugelkopfförmige Gestaltung erstreckt sich vorzugsweise auch über einen Teil der Höhe oder die gesamte Höhe der Ausnehmung.

Vorzugsweise ist die Ausnehmung, an welche sich ein Gewindeabschnitt in Form einer Gewindebohrung anschließen kann, als Durchtrittsöffnung durch den Schraubenkopf ausgebildet.

Die erfindungsgemäße Befestigungseinrichtung, welche der Festlegung eines spinalen Fixiermittels an einer Knochenschraube, insbesondere einer oben beschriebenen Knochenschraube, dient, weist einen im wesentlichen zylindrischen Fortsatz zur Einführung in eine Ausnehmung einer korrespondierenden Knochenschraube auf, wobei der Fortsatz eine Durchtrittsöffnung für ein Verbindungselement zur Festlegung der Befestigungseinrichtung an dem Schraubenkopf aufweist. Ein derartiges Verbindungselement kann insbesondere als Schraubbolzen ausgeführt sein. Unter einer im Wesentlichen zylindrischen Gestalt des Fortsatzes sei auch eine weiter unten beschriebene mehreckige Gestalt oder andere nichtrunde Gestalt verstanden, bei welcher der Fortsatz an einer Innenwandung einer Ausnehmung anlegbare Anlageflächen aufweist, die auf einem Kreisbogen liegen. Ferner soll die im wesentlichen zylindrische Ausgestaltung auch eine konische oder eine andere rotationssymmetrische Form der Fortsatzes bzw. einer Einhüllenden desselben mit umfassen.

Die Befestigungseinrichtung weist einen dem Schraubenschaft zugewandten und einen abgewandten Schenkel auf, zwischen denen ein spinales Fixiermittel festlegbar ist. Es sind aber auch andere Ausgestaltungen der Befestigungseinrichtung möglich. Die Schenkel können einstückig aneinander angeformt sein, beispielsweise in Art einer Klemmschelle, die Schenkel können jedoch auch lösbar aneinander befestigbar sein. Vorzugsweise ist an dem dem Schraubenschaft zugewandten Schenkel der in der Ausnehmung positionierbare Fortsatz des Haltebereichs der Befestigungseinrichtung angeordnet. Gegebenenfalls kann jedoch der Fortsatz auch an dem dem Schraubenschaft abgewandten Schenkel angeordnet sein, welcher den benachbarten Schenkel durchgreift, um in die Ausnehmung einzugreifen. Die Befestigungseinrichtung wird hierbei durch das gleiche Feststellmittel an dem Schraubenkopf befestigt, welches auch der Festlegung des spinalen Fixiermittels dient.

Vorzugsweise liegt der Haltebereich der Befestigungseinrichtung nur im Bereich der Mantelfläche des Fortsatzes an der Kugelkopfschraube an, gegebenenfalls kann auch zusätzlich der die Ausnehmung außenseitig umgebenden umlaufenden Rand des Schraubenkopfes, welcher einen Oberflächenbereich des Schraubenkopfes darstellen kann eine Anlagefläche für den die Befestigungseinrichtung bilden und/oder der dem freien Ende des Fortsatzes gegenüberliegende Bereich der Ausnehmung wie ein zwischen der gewindefreien Ausnehmung und dem Gewindeabschnitt vorgesehener Absatz. Gegebenenfalls kann auch der dem Schraubenkopf zugewandte Schenkel der Befestigungseinrichtung an der Schraubenkopfoberfläche anliegen, beispielsweise an einem Verbindungsbereich des Schenkels mit dem Fortsatz oder über einen darüber hinausgehenden Umfangsbereich, beispielsweise auch vollumfänglich.

Der Fortsatz kann einen runden oder mehreckigen Außenquerschnitt aufweisen, wobei in letzterem Falle die Ausnehmung vorzugsweise einen runden Querschnitt aufweist. Allgemein können der Fortsatz und die Ausnehmung derart ausgeführt, dass der Fortsatz nur mit einem Teil seiner in der Ausnehmung angeordneten Mantelfläche an der Innenwandung der Ausnehmung anliegt. Der Fortsatz kann hierbei drei- bis zwölfeckig oder mehreckig ausgeführt sein, vorzugsweise fünf- bis achteckig. Vorzugsweise ist das Vieleck jeweils gleichseitig, was auch für eine vieleckige Ausgestaltung der Ausnehmung gelten kann. Es versteht sich, dass auch bei einer mehreckigen Ausgestaltung des Fortsatzes bzw. der Ausnehmung diese mehr oder weniger von der Idealform abweichen kann und an den Übergangsbereichen zwischen verschiedenen Flächen Abrundungen vorgesehen sein können oder die Flächen selber eine geringfügige Wölbung, vorzugsweise konkave Wölbung, aufweisen.

Die Anlagebereiche der Ausnehmung oder des Fortsatzes können als leistenartige, sich in Fortsatzlängsrichtung bzw. Ausnehmungslängsrichtung erstreckende Bereiche ausgeführt sein, die mit dem korrespondierenden Anlagebereich des jeweils korrespondierenden Bauteils linienförmig oder flächig zur Anlage bringbar sind. Die leistenartigen Bereiche können insbesondere in den Übergangsbereichen von Außenflächen des Fortsatzes angeordnet sein, die von einem runden Querschnitt stärker abweichen, beispielsweise in dem Übergangsbereich von ebenen Mantelflächen des Fortsatzes bzw. der Ausnehmung. Die leistenartigen Bereiche sind jeweils vorzugsweise gleichmäßig um den Umfang des Fortsatzes oder der Ausnehmung verteilt angeordnet. Die leistenartigen Bereiche können sich um den Umfang des Fortsatzes bzw. der Ausnehmung um weniger als 10°, vorzugsweise weniger als 5° oder um ca. 2° oder weniger erstrecken. Die leistenartigen Bereiche können selber eine im Wesentlichen kreisrunde Außenkontur aufweisen oder aber in Form von im Wesentlichen ebenen Abkantungen von Mantelflächenbereichen des Fortsatzes oder der Ausnehmung bestehen, so dass im Wesentlichen zwei geringfügig beabstandete linienförmige Anlagebereiche bereitgestellt werden, die sich in Längsrichtung der Ausnehmung oder der Fortsatzes erstrecken.

Der dem Fortsatz abgewandte Schenkel der Befestigungseinrichtung kann eine Durchtrittsöffnung zur Durchführung eines Festlegungsmittels der Befestigungseinrichtung an einer korrespondierenden Knochenschraube aufweisen.

Vorzugsweise ist der Mittelpunkt eines Haltebereichs zur Festlegung des spinalen Fixiermittels, wie etwa eines Längsstabes einer Fixiereinrichtung, in etwa auf Höhe der außenseitigen Öffnungsmündung der Ausnehmung oder oberhalb derselben, wie beispielsweise auf Höhe oder oberhalb des Durchstoßpunktes der Ausnehmungslängsachse durch die kugelförmige Einhüllende der Schraubenkopfoberfläche angeordnet. Der Haltebereichmittelpunkt kann in der Höhe in Ausnehmungslängsrichtung um größer/gleich den 1,1 bis 1,2-fachen oder größer/gleich den 1,5-fachen oder 1,75-fachen Kugelkopfradius von dem Kugelkopfmittelpunkt bzw. dem Schnittpunkt der Schraubenschaftlängsachse mit der Ausnehmungslängsachse angeordnet sein.

Der untere Schenkel der Befestigungseinrichtung kann auf einer Kugelabschnittsfläche des Schraubenkopfes aufliegen oder oberhalb dieser abgeordnet sein.

Vorzugsweise ist die Knochenschraube mit zugeordneter und an dieser bestimmungsgemäß festgelegter Befestigungseinrichtung derart ausgebildet, dass in sämtlichen möglichen Sollstellungen der Befestigungseinrichtung die Knochenschraube bis zum Schraubenkopf in einen Knochen, insbesondere Wirbel, eindrehbar ist. Dies gilt insbesondere für eine Sollstellung, in welcher der Haltebereich der Befestigungseinrichtung für ein spinales Fixiermittel einen geringstmöglichen Abstand zu dem dem Schraubenkopf abgewandten Ende des Schraubenschaftes aufweist bzw. sich in der durch die Aufnahmelängsrichtung und die Schraubenschaftlängsrichtung aufgespannten Ebene in der dem Schraubenschaft zugewandten Stellung befindet.

In nicht festgelegtem Zustand kann der Fortsatz gegenüber dem Schraubenkopf um die Ausnehmungslängsachse zumindest um einen Teilumfang wie beispielsweise um 90° oder mehr oder um 180° oder mehr, oder vollumfänglich verdrehbar, insbesondere frei verdrehbar, sein.

Vorzugsweise ist eine Knochenschraube mit zugeordneter Befestigungseinrichtung mit einer Führung versehen, mittels welcher ein Verbindungsmittel wie beispielsweise ein Schraubbolzen zur Festlegung der Befestigungseinrichtung an dem Schraubenkopf durch die Befestigungseinrichtung passgenau an den zugeordneten Gewindeabschnitt des Schraubenkopfes heranführbar ist. Die Anordnung und Festlegung des Verbindungsmittels an dem Schraubenkopf wird hierdurch erleichtert. Diese Führung kann durch den dem Schraubenkopf zugewandten und/oder abgewandten Schenkel, durch den Fortsatz der Befestigungseinrichtung und/oder durch einen anderen geeigneten Bereich der Knochenschraube und/oder der Befestigungseinrichtung bereitgestellt werden. Insbesondere kann eine Durchtrittsöffnung des Fortsatzes vorgesehen sein, die konzentrisch zu dem als Gewindebohrung ausgeführten Gewindeabschnitt ausgeführt ist und unter Berücksichtigung des Gewindes in etwa den gleichen Innendurchmesser aufweist wie die Gewindebohrung.

Die Erfindung sei nachfolgend beispielhaft beschrieben und anhand der Figuren beispielhaft erläutert. Es zeigen:
- Figur 1: eine Darstellung einer Knochenschraube in einer Ansicht von hinten (Fig. 1a), eine Seitenansicht (Fig. 1b), eine Frontansicht (Fig.1c), einen Schnitt A-A gemäß Figur 1b (Fig. 1d,f) und eine perspektivische Ansicht (Fig. 1e),
- Figur 2: eine Befestigungseinrichtung für eine Knochenschraube gemäß Figur 1 in perspektivischer Darstellung (Figur 2a), in Draufsicht (Fig. 2b), in Seitenansicht (Fig. 2c), in Ansicht von unten (Fig. 2d), entlang eines Schnittes A-A gemäß Figur 2b (Fig. 2e) und in einer alternativen Ausführungsform (Fig. 2f).
- Figur 3: eine Knochenschraube gemäß Fig. 1 (teilweise im Ausschnitt) und eine Befestigungseinrichtung gemäß Fig. 2 in verschiedenen Ansichten.

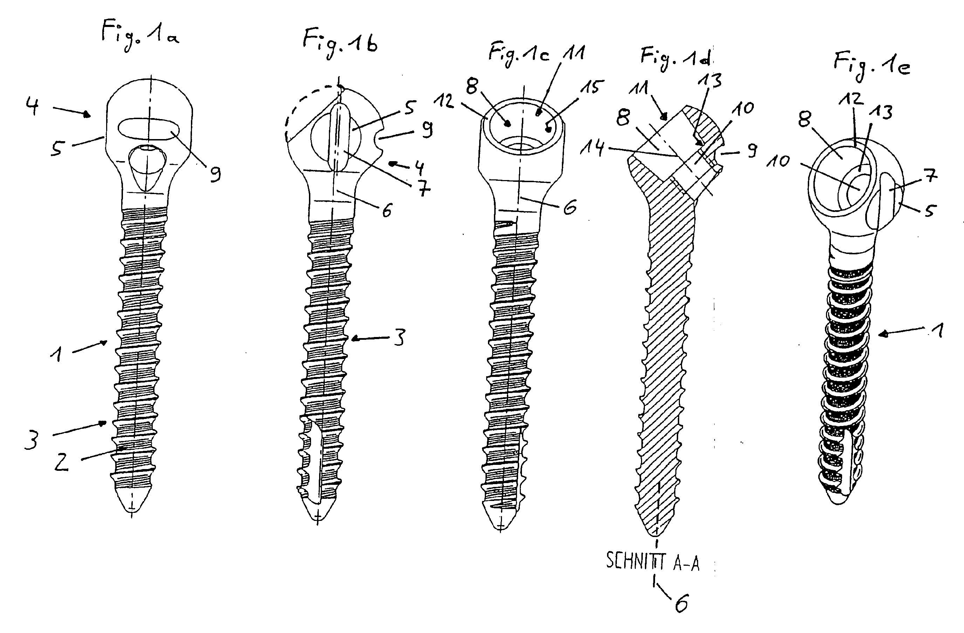

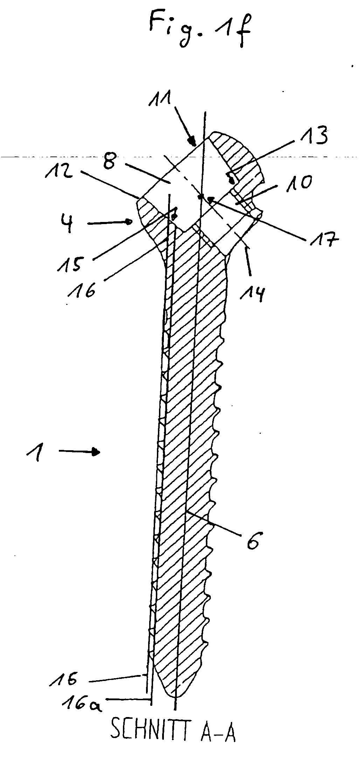

Die Knochenschraube 1 gemäß Figur 1 dient der Anbringung eines spinalen Fixiermittels wie eines Längsstabes und kann insbesondere als Pedikelschraube verwendet werden. Die Knochenschraube kann mit dem mit einem Gewinde 2 versehenen Schraubenschaft 3 in einen Wirbel oder einen anderen Knochen eingeschraubt werden. Der Schraubenkopf 4 ist im Wesentlichen als Kugelkopf ausgeführt, der seitliche Abflachungen 5 aufweist, die mit Haltebereichen für einen nicht dargestellten Verbindungsstab in Form von parallel zur Schaftlängsachse verlaufenden Nuten 7 versehen sind, in welche in bekannter Weise entsprechende Fortsätze des Verbindungsstabes eingreifen können. Ferner ist an der einer Ausnehmung 8 abgewandten Rückseite des Schraubenkopfes ein Haltebereich in Form einer sich quer zur Schaftlängsachse erstreckenden Nut 9 eingebracht, die der Ankoppelung eines alternativ ausgeführten Verbindungsstabes oder einer anderen Einrichtung zur Verwendung mit der erfindungsgemäßen Knochenschraube dient.

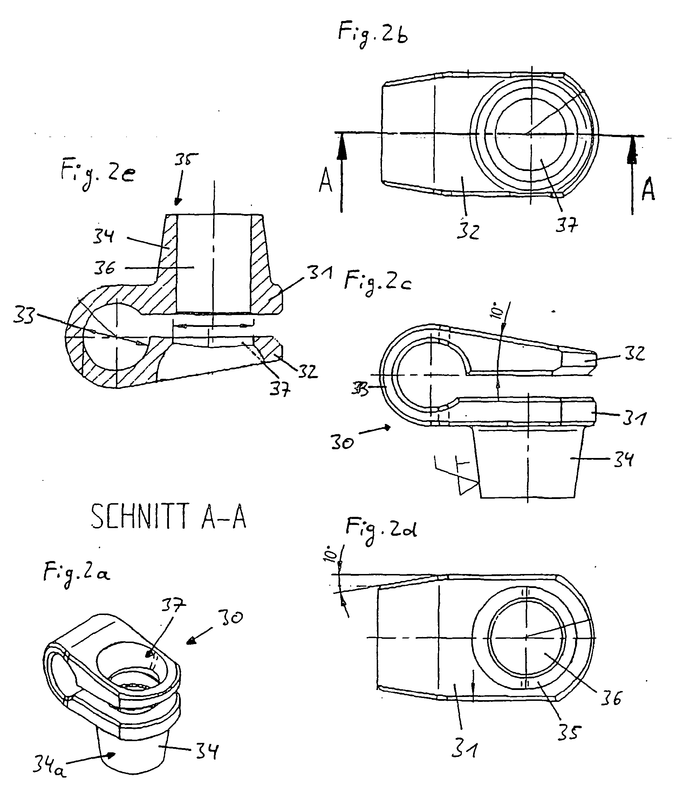

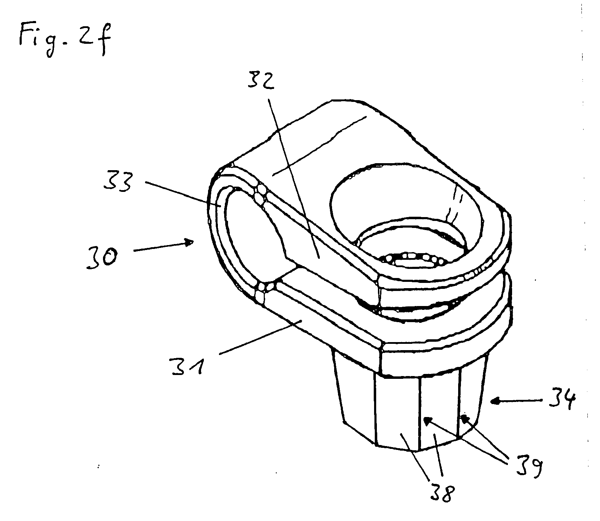

Die im Wesentlichen oder exakt zylindrische und vorzugsweise gewindefreie Ausnehmung 8 dient der Aufnahme eines als Fortsatz 34 ausgebildeten Haltebereichs der in Figur 2 dargestellten Befestigungseinrichtung 30, wobei die Befestigungseinrichtung im nicht festgelegten Zustand frei um die Ausnehmungslängsachse verdrehbar ist. Der im wesentlichen zylindrische Fortsatz wird hierbei seitlich spielfrei und verkippsicher in der Ausnehmung gehaltert. Zur verdrehfesten Festlegung der Befestigungseinrichtung 30 an der Knochenschraube 1 dient ein als Gewindebohrung 10 ausgebildeter Gewindeabschnitt, der einen korrespondierenden Schraubbolzen 41 aufnehmen kann, mittels dessen zugleich der zugeordnete Bestandteil des spinalen Fixiermittels an der Knochenschraube festlegbar ist. Der Schraubbolzen weist hierbei zwischen Schraubenkopf und Gewinde eine umlaufende Nut 42 auf, die zumindest bereichsweise auf Höhe des Spaltes zwischen den beiden Schenkeln der Befestigungseinrichtung abgeordnet ist, nach dem Ausführungsbeispiel befindet sich der Nutgrund auf Höhe der Oberseite des dem Schraubenkopf zugewandten Schenkels. Die Gewindebohrung 10 ist hierbei konzentrisch zu der zylindrischen Ausnehmung 8 angeordnet. Die vorzugsweise gewindefreie Ausnehmung 8 ist hierbei der Schraubenkopfoberseite zugewandt, der Gewindeabschnitt ist in Einführrichtung des Verbindungsmittels nachgelagert bzw. auf der der Einführöffnung 11 der Ausnehmung abgewandten Seite angeordnet. Die Ausnehmung 8 weist über ihre gesamte Tiefe einen größeren Durchmesser auf, als die Gewindebohrung 10. Ausnehmung 8 und Gewindebohrung 10 sind in Art einer Stufenbohrung ausgeführt, wobei diese jeweils an ihrem der Einführöffnung 11 zugewandten Ende durch einen Rand 12 bzw. Absatz 13 außenseitig umgeben werden. Der Rand 12 liegt hierbei in einer Kugelabschnittsebene, welche den kugelförmigen Schraubenkopf abkappt. Der den Gewindeabschnitt radial außenseitig umgebende Absatz 13 weist eine größere radiale Erstreckung auf als der die Ausnehmung radial außen umgebende Absatz bzw. Rand 12.

Die Ausnehmung 8 ist nach dem Ausführungsbeispiel konisch ausgeführt und verjüngt sich zu dem Schraubenkopfmittelpunkt hin. Die Neigung der Innenwandung der Ausnehmung und die Neigung der korrespondierenden Anlageflächen des Fortsatzes entsprechen einander, die Neigung zur Ausnehmungslängsachse beträgt ca. 7°. Der Winkel zwischen der Längsachse 14 der Ausnehmung 8 bzw. des konzentrisch angeordneten Gewindeabschnittes mit der Schraubenschaftlängsachse 6 beträgt ca. 45°.

Die Ausnehmung weist gemäß dem Ausführungsbeispiel einen runden Querschnitt auf, die mit der Innenwandung 15 der Ausnehmung zur Anlage kommende Mantelfläche 34a des Fortsatz 34 ist nach Fig. 2a rotationssymmetrisch bzw. konisch mit gleichem Konuswinkel ausgeführt, so Fortsatz und Ausnehmungswandung vollumfänglich flächig aneinander anlegbar sind. Gemäss der weiteren Ausführungsform nach Fig. 2f weist der im wesentlichen zylindrische Fortsatz 34 einen mehreckigen, sich auf das freie Ende verjüngenden Querschnitt auf. Hierdurch werden leistenförmige Anlagebereiche 39 geschaffen, die jeweils zwischen zurückspringenden, vorzugsweise ebenen Bereichen 38 angeordnet sind und sich in Längsrichtung des Fortsatzes bzw. bei montierter Befestigungseinrichtung im Wesentlichen in Längsrichtung der Ausnehmung erstrecken. Die leistenartigen Anlagebereiche 39 können linienförmig oder flächig mit einem Umfangsbereich an der Ausnehmungsinnenwandung 15 anliegen. Die umfängliche Winkelerstreckung eines jeden der leistenartigen Anlagebereiche 39 kann z.B. ca. 5° betragen, ohne hierauf beschränkt zu sein. Durch die leistenförmigen Anlagenbereiche kann ein Festsetzen der Bauteile aneinander bei Ausübung hoher Anpresskräfte vermieden werden. Die leistenförmigen Anlagebereiche 39 erstrecken sich hierbei über die gesamte Höhe des Fortsatzes 34, ohne dass dies zwingend notwendig ist.

Gemäß Figur 1f erstreckt sich auf der dem Schraubenschaft nächst benachbarten Seite der Ausnehmung 8, welche in der durch die Ausnehmungslängsachse 14 und der Schraubenschaftlängsachse 6 aufgespannten Ebene liegt, die Innenwandung 15 der Ausnehmung in ihrer Höhe bis über die Verlängerung der Mantelfläche 16 des Schraubenschaftes hinaus in den Schraubenkopf hinein. Die Mantelfläche ist hierbei die Einhüllende des Schraubenschaftes einschließlich Gewinde, gegebenenfalls kann sich dies jedoch auch auf die Mantelfläche des Schraubenschaftes bezogen auf die Basis der jeweiligen Gewindegänge beziehen (Mantelfläche 16a). Die Ausnehmung kann sich so sehr weit und unabhängig von der Gestaltung der Außenoberfläche des Schraubenkopfes in diesen erstrecken.

Der die Tiefe der gewindefreien Ausnehmung begrenzende Querschnittsbereich, der hier durch den Absatz 13 bzw. Boden der Ausnehmung gebildet wird, ist bezogen auf die Ausnehmungslängsachse auf der der Öffnung der Ausnehmung abgewandten Seite des Schnittpunktes der Schraubenlängsachse mit der Ausnehmungslängsachse oder auch auf Höhe des Schnittpunktes angeordnet. Dies kann entsprechend auch für das freie Ende des in der Ausnehmung angeordneten Fortsatzes bei festgelegter Befestigungseinrichtung gelten, das aber von dem Boden der Ausnehmung auch beabstandet sein kann. Das Ende der gewindefreien Ausnehmung ist somit auf der der Einführöffnung 11 der Ausnehmung abgewandten Seite des geometrischen Mittelpunktes 17 des Schraubenkopfes angeordnet. Die Ausnehmung 8 und Gewindebohrung 10 sind nach dem Ausführungsbeispiel als Stufenbohrung mit Durchtrittsöffnung 18 ausgeführt, wodurch sich fertigungstechnische Vorteile ergeben.

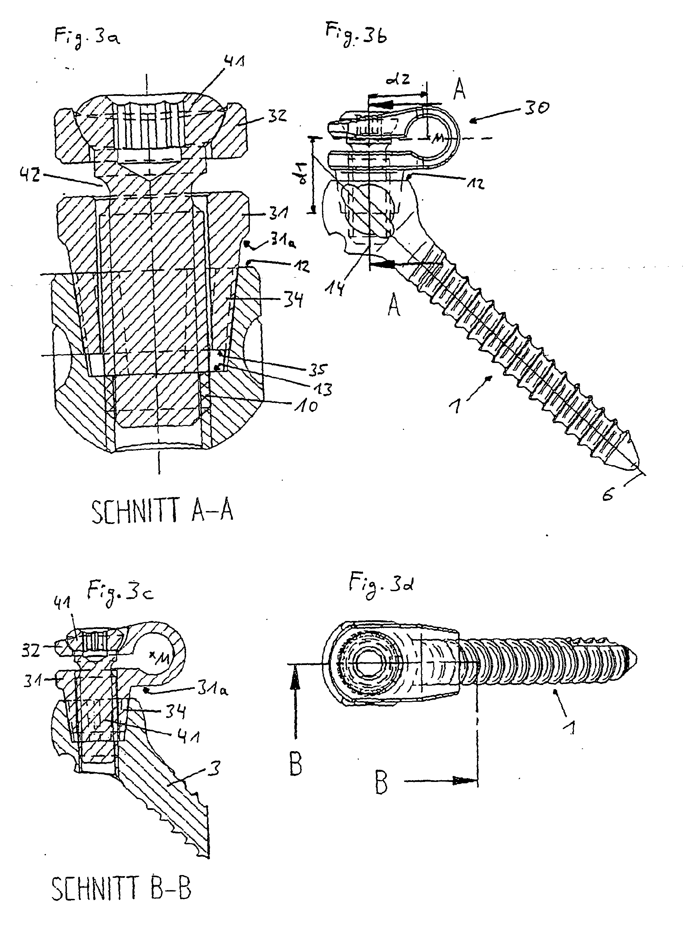

Die in Figur 2 dargestellte Befestigungseinrichtung 30 ist in Art einer Klemmschelle ausgeführt und weist jeweils einen dem Schraubenkopf zugewandten Schenkel 31 ("unterer Schenkel") und einen abgewandten Schenkel 32 ("oberer Schenkel") auf, die durch einen bogenförmigen Haltebereich 33 miteinander verbunden sind, in welchem beispielsweise ein Längsstab einer Fixiereinrichtung festlegbar ist. Der an dem unteren Schenkel 31 angeordnete Fortsatz 34 ist in die Ausnehmung 8 einführbar. Nach dem Ausführungsbeispiel ist dann die dem Schraubenkopf zugewandte Unterseite 31a des Schenkels 31 von dem die Ausnehmung umgebenden Rand 12 in der Höhe beabstandet (siehe Fig. 3a,b). Die freie Stirnseite 35 des Fortsatzes ist hierbei geringfügig von dem die Gewindebohrung 10 außenseitig umgebenden Absatz 13 beabstandet, wodurch sich ein sicherer und definierter Sitz der Befestigungseinrichtung in der Ausnehmung 8 ergibt. Der Fortsatz 34 weist eine zentrische Durchtrittsöffnung 36 auf, die stufenlos von dem Fortsatz in den unteren Schenkel 31 übergeht und die vorzugsweise zylinderförmig ausgeführt ist. Die Durchtrittsöffnung 36 wird durch Einsetzen des Fortsatzes in die Ausnehmung 8 koaxial zu der Gewindebohrung 10 zentriert. Ferner weist der dem Schraubenkopf abgewandte Schenkel 32 eine entsprechende Durchtrittsöffnung 37 auf, deren Durchmesser gleich oder größer als der des unteren Schenkels ist. Die Durchtrittsöffnung 36 dient zugleich als Führung, mittels welcher ein eingeführtes Verbindungsmittel wie der Schraubbolzen 41 passgenau an den Gewindeabschnitt des Schraubenkopfes heranführbar ist, wodurch die Montage der Befestigungseinrichtung wesentlich erleichtert wird. Die Durchtrittsöffnung 36 ist zylinderförmig ausgeführt. Gegebenenfalls können die Durchtrittsöffnungen 37 und/oder 36 sich auch in Richtung auf den jeweiligen Einführbereich hin erweitern.

Gemäß Figur 3b weist der Haltebereich 33 der Befestigungseinrichtung einen Mittelpunkt M auf, welcher durch die Längsachse eines in dem Haltebereich fixierten Rundstabes definiert wird, wobei der Haltebereichmittelpunkt bei montierter Befestigungseinrichtung oberhalb der Höhe des Durchstoßpunktes der Ausnehmungslängsachse 14 durch eine kugelförmige Einhüllende des Schraubenkopfes, wie sie in der Figur 1b schematisch dargestellt ist, angeordnet ist. Hierdurch ergeben sich bei der Ausrichtung des Längsstabes eines spinalen Fixiermittels sowie bei dessen Befestigung besondere Vorteile. Das Verhältnis des Abstandes d1, der der Abstand der durch den Mittelpunkt M verlaufenden, auf der Ausnehmungslängsachse 14 senkrecht stehenden Ebene zu dem Schnittpunkt der Ausnehmungslängsachse 14 zu der Schraubenschaftlängsachse 6 ist, zu dem Abstand d2 des Mittelpunktes M des Haltebereichs zu der Ausnehmungslängsachse beträgt größer/gleich 1,0 bis 1,05, vorzugsweise größer/gleich 1,1 bis 1,25, beispielsweise ca. 1,3 oder größer.

Die Durchführung des Fortsatzes für den Schraubbolzen ist vorzugsweise nicht mit einem Gewinde versehen oder nicht mit einem Gewinde, welches mit dem Schraubbolzen zusammenwirken kann. Vorzugsweise ist die Durchführung glattwandig. Vorzugsweise ist der Fortsatz bei Durchführung des Schraubbolzens und/oder Festlegung des Fortsatzes an dem Schraubenkopf formstabil.

Ferner kann bei der erfindungsgemäßen Anordnung der Befestigungseinrichtung an dem Schraubenkopf mittels eines in den Schraubenkopf eingreifenden Fortsatzes die Knochenschraube auf besonders einfache Weise derart ausgebildet werden, dass diese bei sämtlichen möglichen Sollstellungen der Befestigungseinrichtung bis zum Schraubenkopf in den zugeordneten Knochen eindrehbar ist, wobei stets die Befestigungseinrichtung mit ihrer auf den Schaft zuweisenden Unterseite geringfügig von dem Knochen beabstandet ist. Die Befestigungseinrichtung ist somit praktisch frei um die Ausnehmungslängsachse verdrehbar.

Durch die erfindungsgemäße Knochenschraube mit zugeordneter Befestigungseinrichtung, die mit einem Haltebereich in die Ausnehmung des Schraubenkopfes eingreift, wird somit ermöglicht, dass die Befestigungseinrichtung auch in vormontiertem Zustand sicher an dem Schraubenkopf gehaltert ist, dass auch ohne Verzahnung der Kugelkopfoberfläche stets ein sicherer Halt der Befestigungseinrichtung ermöglicht ist und dass diese hohe Kräfte aufnehmen kann, auch wenn beispielsweise der Längsträger eines spinalen Fixiermittels zur Ausrichtung der entsprechenden Wirbelsäule eines Patienten deformiert werden muss. Ferner ist die Knochenschraube mit Befestigungseinrichtung minimalinvasiv implantierbar.

### Bezugszeichenliste

- 1: Schraube
- 2: Gewinde
- 3: Schaft
- 4: Schraubenkopf
- 5: Abflachung
- 6: Schaftlängsachse
- 7: Nut
- 8: Ausnehmung
- 9: Nut
- 10: Gewindebohrung
- 11: Einführöffnung
- 12: Rand
- 13: Absatz
- 14: Längsachse
- 15: Innenwandung
- 16: Mantelfläche
- 16a: Mantelfläche
- 17: Mittelpunkt
- 18: Durchtrittsöffnung
- 30: Befestigungseinrichtung
- 31: Schenkel
- 31a: Unterseite
- 32: Schenkel
- 33: Haltebereich
- 34: Fortsatz
- 34a: Mantelfläche
- 35: Stirnseite
- 36, 37: Durchtrittsöffnung
- 38: ebener Bereich
- 39: Anlagebereich
- 41: Schraubbolzen
- 42: Nut
- M: Mittelpunkt

## Patentansprüche

1. Knochenschraube (1) zur Anbringung eines spinalen Fixiermittels, insbesondere als Pedikelschraube, mit einem in einem Knochen, insbesondere Wirbel, festlegbaren Schraubenschaft (3) mit Gewinde (2) zum Einschrauben in den Knochen und mit einem Schraubenkopf (4) mit einem Befestigungsbereich für eine Befestigungseinrichtung zur Festlegung eines spinalen Fixiermittels,
- wobei der Befestigungsbereich einen Gewindeabschnitt (10) zur Festlegung der Befestigungseinrichtung aufweist,
- ferner umfassend eine Befestigungseinrichtung (30) mit einem Haltebereich (33) zur Festlegung eines spinalen Fixiermittels und mit einem Befestigungsbereich zur Festlegung an der Knochenschraube,
- wobei der Schraubenkopf (4) eine abschnittsweise gewindefreie Ausnehmung (8) mit einer Längsachse (14) aufweist,
- wobei der Befestigungsbereich der Befestigüngseinrichtung einen in die Ausnehmung (8) der zugeordneten Knochenschraube eingreifenden Fortsatz aufweist,
- wobei die Ausnehmung und der als Gewindebohrung ausgeführte Gewindeabschnitt (10) in Art einer Stufenbohrung ausgeführt sind und wobei die Ausnehmung (8) zur Aufnahme des Fortsatzes (34) bezogen auf den Gewindeabschnitt (10) der Schraubenkopfoberfläche zugewandt angeordnet ist und einen größeren Durchmesser aufweist als dieser,
- wobei der Fortsatz verdrehsicher in der Ausnehmung (8) festlegbar ist und wobei in nicht festgelegte Zustand der Fortsatz gegenüber dem Schraubenkopf um die Ausnehmungslängsachse (14) zumindest um einen Teilumfang verdrehbar ist,
**dadurch gekennzeichnet,**
- **dass** der Fortsatz bei nicht festgelegter Befestigungseinrichtung seitlich spielfrei und verkippsicher in der Ausnehmung gehaltert ist,
- **dass** die Befestigungseinrichtung in Befestigungslage einen dem Schraubenkopf (4) zugewandten und einen diesem abgewandten Schenkel (31, 32) aufweist, zwischen denen ein spinales Fixiermittel festlegbar ist, und
- **dass** die Befestigungseinrichtung durch das gleiche Feststellmittel (41) an dem Schraubenkopf befestigbar ist, mittels welchem das spinale Fixiermittel an der Befestigungseinrichtung festlegbar ist.

2. Knochenschraube nach Anspruch 1, **dadurch gekennzeichnet, dass** die Ausnehmung (8) zur Aufnahme des Fortsatzes (34) im Wesentlichen zylindrisch ausgeführt ist oder einen nichtrunden Querschnitt mit Anlagebereichen zur Halterung des Fortsatzes (34), die auf einer kreisbogenförmigen Einhüllenden liegen, aufweist und dass der Gewindeabschnitt (10) im Wesentlichen konzentrisch zu der Ausnehmung (8) angeordnet ist.

3. Knochenschraube nach einem der Ansprüche 1 oder 2, **dadurch gekennzeichnet, dass** die Ausnehmung (8) sich zum Inneren des Schraubenkopfes (4) hin verjüngt.

4. Knochenschraube nach einem der Ansprüche 1 bis 3, **dadurch gekennzeichnet dass** der den Gewindeabschnitt (10) radial außenseitig umgebende Absatz (13) eine größere radiale Erstreckung aufweist als der die Ausnehmung (8) radial außen umgebende Rand (12).

5. Knochenschraube nach einem der Ansprüche 1 bis 4, **dadurch gekennzeichnet, dass** sich die Innenwandung der Ausnehmung (8) in der Höhe auf der dem Schraubenschaft (3) nächst benachbarten Seite der Ausnehmung (8) bis über die Verlängerung der Mantelfläche (16,16a) des Schraubenschaftes (3) hinaus in den Schraubenkopf hinein erstreckt.

6. Knochenschraube nach einem der Ansprüche 1 bis 5, **dadurch gekennzeichnet, dass** der die Tiefe der gewindefreien Ausnehmung (8) begrenzende Querschnittsbereich in etwa auf Höhe oder auf der der Öffnung der Ausnehmung (8) abgewandten Seite des Schnittpunktes der Schraubenlängsachse (6) mit der Ausnehmungslängsachse liegt.

7. Knochenschraube nach einem der Ansprüche 1 bis 6, **dadurch gekennzeichnet, dass** der Schraubenkopf (4) im Wesentlichen als Kugelkopf oder Teilkugelkopf ausgeführt ist.

8. Knochenschraube nach einem der Ansprüche 1 bis 7, **dadurch gekennzeichnet, dass** die Ausnehmung (8) als Durchtrittsöffnung durch den Schraubenkopf (4) ausgeführt ist.

9. Knochenschraube nach einem der Ansprüche 1 bis 8, **dadurch gekennzeichnet, dass** der Fortsatz (34) der Befestigungseinrichtung im Wesentlichen zylindrisch ausgeführt ist und eine Durchtrittsöffnung (18) für ein Verbindungselement (40) zur Festlegung der Befestigungseinrichtung (30) an dem Schraubkopf (4) aufweist.

10. Knochenschraube nach einem der Ansprüche 1 bis 9, **dadurch gekennzeichnet, dass** der Fortsatz an dem dem Schraubenkopf (4) zugewandten Schenkel angeordnet ist.

11. Knochenschraube nach einem der Ansprüche 1 bis 10, **dadurch gekennzeichnet, dass** der Fortsatz (34) sich in Richtung auf sein freies Ende hin verjüngt.

12. Knochenschraube nach einem der Ansprüche 1 bis 11, **dadurch gekennzeichnet, dass** der in der Ausnehmung (8) angeordnete Fortsatz (34) bei festgelegter Befestigungseinrichtung (30) nur mit Teilbereichen seiner in der Ausnehmung (8) angeordneten Mantelfläche an der Innenwandung der Ausnehmung (8) anliegt.

13. Knochenschraube nach einem der Ansprüche 1 bis 12, **dadurch gekennzeichnet, dass** der Mittelpunkt des Haltebereichs (33) zur Festlegung des spinalen Fixiermittels in etwa auf Höhe oder oberhalb einer den Durchstoßpunkt der Ausnehmungslängsachse (14) durch die Kugeloberfläche aufweisenden, senkrecht zur Ausnehmungslängsachse (8) angeordneten Ebenen des Schraubenkopfes (4) angeordnet ist.

14. Knochenschraube nach einem der Ansprüche 1 bis 13, **dadurch gekennzeichnet, dass** an der Befestigungseinrichtung eine Führung (36,37) für ein Verbindungsmittel zur Festlegung der Befestigungseinrichtung (30) an dem Schraubenkopf (4) vorgesehen ist, und dass mittels der Führung das Verbindungsmittel passgenau an den Gewindeabschnitt (10) des Schraubenkopfes (4) heranführbar ist.

15. Knochenschraube nach einem der Ansprüche 1 bis 14, **dadurch gekennzeichnet, dass** im festgelegten Zustand der Befestigungseinrichtung (30) das dem Haltebereich für das spinale Fixiermittel abgewandte Ende des Fortsatzes von dem Ende der gewindefreien Ausnehmung (8) beabstandet ist und/oder dass der Fortsatz (34) mit seinem dem Haltebereich für das spinale Fixiermittel zugewandten Ende von der gewindefreien Ausnehmung (8) vorsteht.

## Claims

1. Bone screw (1) for attaching a spinal fixing element, especially as a pedicle screw, with a screw shank (3) that can be fixed in place in a bone, especially a vertebra, with a thread (2) for screwing into the bone, and with a screw head (4) with a fastening area for a fastening device for fixing a spinal fixing element in place,
- where the fastening area displays a threaded section (10) for fixing the fastening device in place,
- further encompassing a fastening device (30) with a retaining area (33) for fixing the spinal fixing element in place and with a fastening area for fixing in place on the bone screw,
- the screw head (4) displays a recess (8) with a longitudinal axis (14), sections of which are threadless, wherein the fastening area of the fastening device displays an extension engaging the recess (8) of the associated bone screw,
- the threaded section (10) is designed as a threaded hole and the recess and the threaded hole are designed in the manner of a stepped hole, and wherein referred to the threaded section (10), the recess (8) for accommodating the extension (34) is located facing towards the surface of the screw head and displays a larger cross-section than the threaded section,
- wherein the extension can be fixed in place in nonrotating fashion in the recess (8), and wherein, when not fixed in place, the extension is rotatable about the longitudinal axis (14) of the recess relative to the screw head, at least over part of the circumference,
**characterized in that**
- when the fastening device is not fixed, the extension is retained in the recess without lateral play and in non-skewing fashion,
- in fastened position the fastening device comprises one leg (31) facing towards the screw head (4) and one leg (32) facing away from the screw head (4) between which a spinal fixing element can be fixed in place, and
- the fastening device is fastened on the screw head by the same fastening means (41) as also used for fixing the spinal fixing element in place at the fastening device.

2. Bone screw according to Claim 1, **characterized in that** the recess (8) for accommodating the extension (34) is of essentially cylindrical design, or displays a non-circular cross-section with contact areas for retaining the extension (34) that lie on an envelope with the shape of an arc of a circle, and **in that** the threaded section (10) is located essentially concentrically to the recess.

3. Bone screw according to any of claims 1 or 2, **characierized in that** the recess (8) tapers towards the inside of the screw head (4).

4. Bone screw according to any of claims 1 to 3, **characterized in that** the shoulder (13) radially surrounding the threaded section (10) on the outside displays a larger radial extension that the edge (12) radially surrounding the recess (8) on the outside.

5. Bone screw according to any of claims 1 to 4, **characterized in that** the height of the inner wall of the recess (8) extends, on the side of the recess (8) most closely adjacent to the screw shank (3), beyond the extension of the lateral surface (16, 16a) of the screw shank (3), into the screw head.

6. Bone screw according to any of claims 1 to 5, **characterized in that** the cross-sectional area limiting the depth of the threadless recess (8) is located roughly at the level of, or on, the side of the intersection of the longitudinal axis (6) of the screw and the longitudinal axis of the recess (8) facing away from the opening of the recess.

7. Bone screw according to any of claims 1 to 6, **characterized in that** the screw head (4) is essentially designed as a spherical head or a partially spherical head.

8. Bone screw according to any of claims 1 to 7, **characterized in that** the recess (8) is designed as a through-hole passing through the screw head (4).

9. Bone screw according to any of claims 1 to 8, **characterized in that** the extension (34) of the fastening device is of essentially cylindrical design and displays a through-hole (18) for a connecting element (40) for fixing the fastening device (30) in place on the screw head (4).

10. Bone screw according to any of claims 1 to 9, **characterized in that** the extension is located on the leg facing towards the screw head (4).

11. Bone screw according to any of claims 1 to 10, **characterized in that** the extension (34) tapers towards its free end.

12. Bone screw according to any of claims 1 to 11, **characterized in that**, when the fastening device (30) is fixed in place, only partial areas of the lateral surface of the extension (34) located in the recess (8) are in contact with the inner wall of the recess (8).

13. Bone screw according to any of claims 1 to 12, **characterized in that** the mid-point of the retaining area (33) for fixing the spinal fixing element in place is located roughly at the level of, or above, a plane of the screw head that displays the piercing point of the longitudinal axis (14) of the recess through the surface of the sphere and is located perpendicular to the longitudinal axis (8) of the recess.

14. Bone screw according to any of claims 1 to 13, **characterized in that** the fastening device is provided with a guide (36, 37) for a connecting means for fixing the fastening device (30) in place on the screw head (4), and **in that** the connecting means can be accurately guided to the threaded section (10) of the screw head (4) by means of the guide.

15. Bone screw according to any of claims 1 to 14, **characterized in that**, when the fastening device (30) is fixed in place, the end of the extension facing away from the retaining area for the spinal fixing element is a distance away from the end of the threadless recess (8), and/or **in that** the end of the extension (34) facing towards the retaining area for the spinal fixing element projects from the threadless recess (8).

## Revendications

1. Vis osseuse (1) pour la mise en place d'un moyen de fixation vertébral, en particulier en tant que vis pédiculaire, avec une tige de vis (3) pouvant être fixée dans un os, notamment des vertèbres, avec un filetage (2) pour vissage dans l'os, et avec une tête de vis (4), avec une zone de fixation pour un dispositif de fixation pour bloquer un moyen de fixation vertébral, la zone de fixation présentant un segment fileté (10) pour le blocage du dispositif de fixation, comprenant, en outre, un dispositif de fixation (30) avec une zone de retenue (33) pour bloquer un moyen de fixation vertébral et avec une zone de fixation pour blocage au niveau de la vis osseuse, la tête de vis (4) présentant un évidement (8) sans filetage par endroit, muni d'un axe longitudinal (14), la zone de fixation du dispositif de fixation présentant un élément de prolongement venant en engagement dans l'évidement (8) de la vis osseuse associée, l'évidement et le segment fileté (10) réalisé en tant que perçage fileté étant réalisés à la manière d'un perçage échelonné et l'évidement (8) étant disposé orienté vers la surface de la tête de vis, par rapport au segment fileté (10), pour la réception de l'élément de prolongement (34) et présentant un diamètre supérieur à celui-ci, l'élément de prolongement pouvant être bloqué, avec blocage en rotation, dans l'évidement (8) et l'élément de prolongement, à l'état non bloqué, étant avec possibilité de rotation par rapport à la tête de vis autour de l'axe longitudinal (14) de l'évidement au moins sur une partie de la périphérie, **caractérisée en ce que** l'élément de prolongement est maintenu latéralement sans jeu et protégé contre le basculement dans l'évidement lorsque le dispositif de fixation n'est pas bloqué, **en ce que** le dispositif de fixation présente à l'état de fixation une aile (31) orientée vers la tête de vis (4) et une aile (32) opposée à celle-ci, entre lesquelles un moyen de fixation vertébral peut être bloqué, et **en ce que** le dispositif de fixation peut être fixé par le même moyen de blocage (41) au niveau de la tête de vis, permettant de bloquer le moyen de fixation vertébral au niveau du dispositif de fixation.

2. Vis osseuse selon la revendication 1, **caractérisée en ce que** l'évidement (8) est réalisé essentiellement cylindriquement pour recevoir l'élément de prolongement (34) ou présente une section non arrondie avec des zones de positionnement pour maintien de l'élément de prolongement (34) qui sont sur une enveloppe en arc de cercle, et **en ce que** le segment fileté (10) est disposé essentiellement concentriquement à l'évidement (8).

3. Vis osseuse selon l'une quelconque des revendications 1 ou 2, **caractérisée en ce que** l'évidement (8) se réduit vers l'intérieur de la tête de vis (4).

4. Vis osseuse selon l'une quelconque des revendications 1 à 3, **caractérisée en ce que** l'épaulement (13) entourant extérieurement et radialement le segment fileté (10) présente un développement radial supérieur au bord (12) entourant à l'extérieur et radialement l'évidement (8).

5. Vis osseuse selon l'une quelconque des revendications 1 à 4, **caractérisée en ce que** la paroi intérieure de l'évidement (8) s'étend, dans la tête de vis, en hauteur, sur le côté, immédiatement contigu à la tige de vis (3), de l'évidement (8), jusqu'au-delà du prolongement de la surface enveloppe (16, 16a) de la tige de vis (3).

6. Vis osseuse selon l'une quelconque des revendications 1 à 5, **caractérisée en ce que** la zone de section délimitant la profondeur de l'évidement (8) sans filetage, est à peu près à la hauteur ou sur le côté, opposé de l'ouverture de l'évidement (8), du point d'intersection de l'axe longitudinal de la vis (6) avec l'axe longitudinal de l'évidement.

7. Vis osseuse selon l'une quelconque des revendications 1 à 6, **caractérisée en ce que** la tête de vis (4) est réalisée essentiellement en tant que rotule ou rotule partielle.

8. Vis osseuse selon l'une quelconque des revendications 1 à 7, **caractérisée en ce que** l'évidement (8) est réalisé en tant qu'ouverture de passage à travers la tête de vis (4).

9. Vis osseuse selon l'une quelconque des revendications 1 à 8, **caractérisée en ce que** l'élément de prolongement (34) du dispositif de fixation est réalisé essentiellement cylindriquement et présente une ouverture de passage (18) pour un élément de liaison (40) pour bloquer le dispositif de fixation (30) au niveau de la tête de vis (4).

10. Vis osseuse selon l'une quelconque des revendications 1 à 9, **caractérisée en ce que** l'élément de prolongement est disposé au niveau de l'aile orientée vers la tête de vis (4).

11. Vis osseuse selon l'une quelconque des revendications 1 à 10, **caractérisée en ce que** l'élément de prolongement (34) se réduit en direction de son extrémité libre.

12. Vis osseuse selon l'une quelconque des revendications 1 à 11, **caractérisée en ce que**, lorsque le dispositif de fixation (30) est bloqué, l'élément de prolongement (34) disposé dans l'évidement (8) s'applique contre la paroi intérieure de l'évidement (8) uniquement avec des zones partielles de sa surface enveloppe disposée dans l'évidement (8).

13. Vis osseuse selon l'une quelconque des revendications 1 à 12, **caractérisée en ce que** le centre de la zone de retenue (33) est disposé, pour bloquer le moyen de fixation vertébral, à peu près à la hauteur ou au-dessus d'un plan, disposé perpendiculairement à l'axe longitudinal de l'évidement (8), présentant le point de percée de l'axe longitudinal (14) de l'évidement à travers la surface de la rotule, de la tête de vis (4).

14. Vis osseuse selon l'une quelconque des revendications 1 à 13, **caractérisée en ce qu'**il est prévu, au niveau du dispositif de fixation, un guidage (36, 37) pour un moyen de liaison, pour bloquer le dispositif de fixation (30), au niveau de la tête de vis (4) et **en ce qu'**à l'aide du guidage, le moyen de liaison peut être rapproché, en ajustement précis, du segment fileté (10) de la tête de vis (4).

15. Vis osseuse selon l'une quelconque des revendications 1 à 14, **caractérisée en ce que**, le dispositif de fixation (30) à l'état bloqué, l'extrémité, opposée à la zone de retenue pour le moyen de fixation vertébral, de l'élément de prolongement est espacée de l'extrémité de l'évidement sans filetage (8), et/ou **en ce que** l'élément de prolongement (34) dépasse avec son extrémité orientée vers la zone de retenue pour le moyen de fixation vertébral, de l'évidement sans filetage (8).
